# EUROPÄISCHE PATENTSCHRIFT

(11) **EP 1 171 944 B1**
(45) Veröffentlichungstag und Bekanntmachung des Hinweises auf die Patenterteilung: **04.02.2004**
(21) Anmeldenummer: 00922662.2
(22) Anmeldetag: 20.04.2000
(51) Int. Cl.: H02K 7/09, H02K 7/14, A61M 1/10, G01P 5/02, F16C 39/06

(54) **ROTOREINRICHTUNG**
ROTOR DEVICE
DISPOSITIF A ROTOR

(30) Priorität: 20.04.1999 DE 29907332 U; 18.09.1999 DE 19944863
(43) Veröffentlichungstag der Anmeldung: 16.01.2002
(73) Patentinhaber: Forschungszentrum Jülich GmbH, 52425 Jülich (DE)
(72) Erfinder: FREMEREY, Johan, K., D-53127 Bonn (DE)
(74) Vertreter: Paul, Dieter-Alfred, Dipl.-Ing.
(86) Internationale Anmeldenummer: PCT/EP2000/003640
(87) Internationale Veröffentlichungsnummer: WO 2000/064031

(56) Entgegenhaltungen:
- EP-A- 0 856 666
- EP-A- 0 882 427
- GB-A- 2 057 590
- US-A- 3 623 835
- US-A- 4 398 773
- US-A- 4 812 694
- US-A- 5 126 610
- US-A- 5 211 546
- US-A- 5 385 581

## Beschreibung

Die Erfindung betrifft eine Rotoreinrichtung zur Interaktion eines Rotors mit einem Fluid, beispielsweise für eine Pumpe oder ein Meßgerät, wobei unter einem Fluid sowohl ein Gas als auch eine Flüssigkeit zu verstehen ist.

Im Stand der Technik sind für Pumpen und Meßgeräte Rotoreinrichtungen entwickelt worden, bei denen ein Rotor innerhalb eines Trägerrohrs durch magnetische Feldkräfte in einer Gleichgewichtslage gehalten wird. So ist in der DE-A-29 19 236 ein Turbinenradzähler zur Durchflußmessung von Flüssigkeiten beschrieben, bei dem der Rotor für die radiale Stabilität zwei beabstandete, als Permanentmagnete ausgebildete Rotormagnete aufweist, denen paarweise ebenfalls als Permanentmagnete ausgebildete Statormagnete zugeordnet sind, die das Trägerrohr umgeben. Dabei sind Rotor- und Statormagnete in axialer Richtung einander abstoßend magnetisiert.

Zwischen den Statormagneten ist eine elektrische Magnetspule angeordnet, die das Trägerrohr ringförmig umgibt. Die Magnetspule wirkt mit einem ferromagnetischen Flußleitstück am Rotor zusammen, das zwischen den Rotormagneten angeordnet ist. Zusätzlich ist ein Sensor vorhanden, der die Axialposition des Rotors erfaßt und mit einer Regeleinrichtung zusammenwirkt, die den elektrischen Stromfluß in der Magnetspule steuert. Sobald die Feldkräfte der Rotor- und Statormagnete bei einer axialen Verschiebung des Rotors bestrebt sind, den Rotor aus der Gleichgewichtslage heraus in Axialrichtung zu beschleunigen, erzeugt die durch den Sensor gemessene Axialverschiebung des Rotors ein Signal, das in der Magnetspule eine entgegengesetzte stabilisierende Feldkraft bewirkt. Der Rotor wird also bei einer axialen Lageverschiebung in die eine oder die andere Richtung ständig in seine Sollage zurückgeführt. Dabei sind die stabilisierenden Axialkräfte gegenüber der axialen Lageverschiebung zeitlich in bekannter Weise derart phasenverschoben, daß den Rotor sowohl rückstellende als auch dämpfende Kräfte in seiner Sollage stabilisieren.

Ein Nachteil der vorbeschriebenen Rotoreinrichtung besteht darin, daß der Rotor nur eine relativ geringe Lagersteifigkeit in radialer Richtung hat. Die Ursache hierfür ist der große Abstand zwischen den Stator- und Rotormagneten aufgrund des zwischen Trägerrohr und Rotor vorhandenen Ringkanals für die Durchführung des Fluids.

In der DE-A-24 44 099 ist ein Magnetlager für schnell bewegte Körper beschrieben. Dieses Magnetlager weist einen hülsenförmigen Rotor auf, dessen Stirnseiten Permanentmagnete aufweisende Polstücke gegenüberstehen, aufgrund deren Anziehungskräfte der Rotor in einer stabilen Lage gehalten wird. Mittels einer berührungslosen Lageabtastung können Abweichungen von der Gleichgewichtslage festgestellt werden. Solche Abweichungen werden durch eine leistungslose elektromagnetische Streufeldsteuerung ausgeglichen, wobei hierfür ringförmige Spulen vorgesehen sind, die an den Polstücken nahe den Spalten zu dem Rotor angeordnet sind. Ein solches Magnetlager ist für die Anordnung in einem Trägerrohr, durch das ein Fluid geleitet wird, aus räumlichen Gründen nicht geeignet.

Soweit es Pumpen angeht, werden Magnetlager insbesondere für Blutpumpen eingesetzt. So ist aus der US-A-5,695,471 eine Blutpumpe bekannt, die als Radialpumpe mit einem Radialrotor ausgebildet ist. Der Radialrotor ist innerhalb eines Trägerrohrs angeordnet und weist in einem eingangsseitigen Fortsatz eine Mehrzahl von Rotormagneten auf, denen am Trägerrohr Statormagnete zugeordnet sind. Zusätzlich weist der Radialrotor über den Umfang verteilt eine Vielzahl von sich in axialer Richtung erstreckenden, stabförmigen Rotormagneten auf, denen beidseits des Radialrotors auf Seiten des Trägerrohrs ringförmige Statormagnete zugeordnet sind. Diese Rotor- und Statormagnete sollen die Radiallagerung im Bereich des Fortsatzes des Rotors unterstützen. In Axialrichtung ist der Rotor rein mechanisch einer Ends an einer Kugel und anderen Ends an einer Spitzenlagerung gehalten.

Der Rotor wird mittels eines bürstenlosen Drehfeldmotors angetrieben. Hierzu ist auf Seiten des Trägerrohrs eine Spule angeordnet, die mit in den Radialrotor eingelassenen Polradmagneten zusammenwirkt.

Nachteilig bei dieser Blutpumpe ist, daß die Lagerstabilität in radialer Richtung nicht optimal ist und daß die Pumpe wegen der Vielzahl der Rotor- und Statormagnete einen großen Raumbedarf und hohes Gewicht hat. Außerdem unterliegt die rein mechanische Lagerung in axialer Richtung dem Verschleiß, was insbesondere bei implantierbaren Blutpumpen von Nachteil ist.

Als Blutpumpen sind auch axiale Pumpen bekannt. Hier erfolgt die Lagerung jedoch ausschließlich mechanisch in Leiträdern, die ortsfest in dem Trägerrohr vor und hinter dem Rotor angeordnet sind. (Wernicke et al., A Fluid Dynamic Analysis Using Flow Visualization of the Baylor/NASA Implantable Axial Flow Blood Pump for Design Improvemen, Artificial Organs 19(2), 1995, S. 161-177). Solche mechanischen Lagerungen sind verschleißanfällig und haben zudem einen ungünstigen Einfluß auf empfindliche Flüssigkeiten, insbesondere auf Körperflüssigkeiten wie Blut.

Die EP-A- 0 882 427 und EP-A- 0 856 666 offenbaren auch eine gattungsgemäße Rotoreinrichtung.

Der Erfindung liegt die Aufgabe zugrunde, eine Rotoreinrichtung der eingangs genannten Art so zu gestalten, daß eine wesentlich höhere Lagersteifigkeit insbesondere in radialer Richtung erzielt wird und sie sich deshalb sehr vielseitig einsetzen läßt.

Diese Aufgabe wird erfindungsgemäß durch folgende Merkmale gelöst:
a) die Rotoreinrichtung hat ein Trägerrohr;
b) in dem Trägerrohr ist ein Rotor drehbar gelagert;
c) der Rotor ist für die Interaktion mit dem durch das Trägerrohr strömenden Fluid ausgebildet;
d) der Rotor weist an beiden Stirnseiten axial magnetisierte permanentmagnetische Rotormagnete auf:
e) den Stirnseiten des Rotors axial unmittelbar gegenüberliegend sind mit dem Trägerrohr verbundene, permanentmagnetische Statormagnete angeordnet;
f) jeder Statormagnet hat eine solche axiale Magnetisierung, so daß sich benachbarte Stator- und Rotormagnete gegenseitig anziehen.
g) die Rotoreinrichtung weist eine magnetische Axialstabilisiereinrichtung für den Rotor auf.

Alternativ dazu wird die Aufgabe durch eine Rotoreinrichtung mit folgenden Merkmalen gelöst:
a) die Rotoreinrichtung hat ein Trägerrohr;
b) in dem Trägerrohr ist ein Rotor drehbar gelagert;
c) der Rotor ist für die Interaktion mit dem in dem Trägerrohr befindlichen Fluid ausgebildet;
d) an den Stirnseiten des Rotors stehen sich jeweils unmittelbar ein axial magnetisierter, permanent magnetischer Magnet und ein Flußleitstück gegenüber, wobei der Magnet entweder als Rotormagnet am Rotor sitzt oder als Statormagnet mit dem Trägerrohr verbunden ist;
e) die Rotoreinrichtung weist eine magnetische Axialstabilisiereinrichtung auf.

Grundgedanke der Erfindung ist es also, mittels Rotorund Statormagneten in endseitiger Anordnung jeweils ein die Spalte zwischen Rotor und Statormagneten überbrückendes Magnetfeld in axialer Richtung zu erzeugen, das die jeweils gegenüberliegende Paare von Rotormagneten und Statormagneten gegenseitig anzieht. Hierdurch wird die Lagersteifigkeit bei gleicher Geometrie gegenüber dem Magnetlager nach der DE-A-29 19 236 um mindestens eine Zehnerpotenz erhöht, ohne daß hierdurch der Ringkanal zwischen Trägerrohr und Rotornabe wesentlich beeinträchtigt wird.

Der vorbeschriebene Effekt tritt auch schon dann ein, wenn sich nicht zwei Magnete, also Rotor- und Statormagnete, gegenüberstehen, sondern jeweils ein Magnet auf der einen Seite und ein Flußleitstück auf der anderen Seite. Dabei kann alternativ der Magnet als Rotormagnet am Rotor sitzen und das Flußleitstück mit dem Trägerrohr verbunden sein oder das Flußleitstück am Rotor angeordnet sein und der Magnet als Statormagnet am Trägerrohr sitzen. Zur Erzielung einer hohen Lagersteifigkeit können zusätzlich elektrische Magnetspulen zur Verstärkung der Magnetisierung der Flußleitstücke im Sinne einer Vergrößerung der Anziehungskräfte zwischen den Magneten und den Flußleitstücken vorgesehen sein.

Soweit sich jeweils paarweise Rotor- und Statormagnete gegenüberstehen, sollten sie vorzugsweise aus mindestens zwei ineinandergefügten Teilmagneten bestehen, wobei jeweils radial benachbarte Teilmagnete entgegengesetzt magnetisiert sind. Durch diese Ausbildung der Rotor- und Statormagnete wird sogar eine Steigerung der Lagersteifigkeit um den Faktor 40 erzielt.

Der Rotor ist zweckmäßigerweise als Axialrotor ausgebildet, so daß sich bei einer Verwendung der Rotoreinrichtung für eine Pumpe eine Axialpumpe ergibt. Ein solcher Axialrotor ist wesentlich weniger aufwendig zu gestalten als ein Radialrotor.

Nach der Erfindung ist ferner vorgesehen, daß der Rotor eine Rotornabe aufweist und die Rotormagnete bzw. Flußleitstücke in der Rotornabe angeordnet sind, wobei die Statormagnete bzw. Flußleitstücke den Stirnseiten der Rotornabe gegenüberliegend angeordnet sind. Die Statormagnete bzw. Flußleitstücke können über strömungsgünstig ausgebildete Stege mit dem Trägerrohr verbunden sein. Durch diese Anordnung ergibt sich eine kompakte Konstruktion, und es werden schädliche Spalte weitestgehend vermieden. Dabei sollten die Statormagnete bzw. Flußleitstücke in Radialstabilisatoren angeordnet sein, deren Kontur nicht über die der Rotornabe vorsteht, wobei die Radialstabilisatoren vorzugsweise die gleiche Kontur haben sollten wie die Rotornabe.

In weiterer Ausbildung der Erfindung ist vorgesehen, daß von den jeweils gegenüberliegenden Stirnseiten der Radialstabilisatoren und des Rotors zumindest jeweils eine sphärisch ausgebildet ist. Durch diese Ausbildung wird ein mechanisches Anlaufen von achsfernen Bereichen des Rotors und der Radialstabilisatoren bei axialer Auslenkung des Rotors vermieden. Zur Begrenzung der Radialund/oder Axialbeweglichkeit des Rotors ist es zweckmäßig, daß die jeweils gegenüberliegenden Stirnflächen der Radialstabilisatoren und des Rotors mit ineinandergreifenden komplementären Lagerzapfen und Lagerausnehmungen versehen sind, wobei durch ein entsprechendes radiales Spiel sichergestellt wird, daß Lagerzapfen und Lagerausnehmungen sich nur bei relativ großen Auslenkungen des Rotors in radialer Richtung berühren.

In weiterer Ausgestaltung der Erfindung ist vorgesehen, daß die Rotormagnete und die Statormagnete jeweils unmittelbar gegenüberliegend angeordnet sind, damit ein möglichst starkes Magnetfeld entsteht.

Nach der Erfindung ist ferner vorgeschlagen, daß die Axialstabilisiereinrichtung wenigstens eine elektrische Magnetspule sowie eine Regeleinrichtung mit einem die Axialbewegung des Rotors erfassenden Sensor aufweist, wobei die Regeleinrichtung den elektrischen Stromfluß in der Magnetspule bzw. den Magnetspulen derart beeinflußt, daß das Magnetfeld der Magnetspule(n) einer Axialbewegung des Rotors aus seiner Sollage entgegenwirkt. Eine solche Axialstabilisiereinrichtung ist prinzipiell schon aus der DE-A 29 19 236 und auch aus der DE-A-24 44 099 bekannt und hat sich bewährt. Zweckmäßigerweise sollte die Axialstabilisiereinrichtung zwei Magnetspulen aufweisen, die im Bereich der Statormagnete und/oder den Stirnflächen des Rotors angeordnet sind, damit die Axialstabilisierung besonders wirksam ist.

Dabei bestehen zweierlei Möglichkeiten für die Anordnung der Magnetspulen, nämlich zum einen am Trägerrohr in der Weise, daß sie das Trägerrohr umgeben, zum anderen in den Radialstabilisatoren selbst, wobei jedoch dann für die Herausführung der Leitungen zu und von den Magnetspulen gesorgt werden muß. Vorzugsweise sollten die Radialstabilisatoren magnetsierbare Flußleitstücke in einer solchen Ausbildung und Anordnung aufweisen, daß das von Rotorund Statormagneten erzeugte axiale Magnetfeld im Spalt zwischen den Stirnseiten von Radialstabilisatoren und Rotor durch das von den Magnetspulen erzeugte Magnetfeld in axialer Richtung überlagert wird, und zwar in der Weise, daß einer Axialbewegung des Rotors aus der Sollage entgegengewirkt wird. Dabei können die Magnetspulen selbst als Sensoren verwendet werden. Die Flußleitstücke sind zweckmäßigerweise auf Höhe der Magnetspulen angeordnet.

Soweit die Rotoreinrichtung Teil einer Meßeinrichtung sein soll, ist es zweckmäßig, daß der Rotor einen Impulsgeber und das Trägerrohr einen Impulsnehmer aufweist und daß der Impulsgeber für den Impulsnehmer der Drehzahl des Rotors entsprechende Impulse erzeugt. Dies kann in einfacher weise dadurch geschehen, daß der Impulsgeber als Impulsmagnet(e) und der Impulsnehmer als Spule ausgebildet ist, so daß in der Spule beim Drehen des Rotors ein elektrischer Strom induziert wird.

In weiterer Ausgestaltung der Erfindung ist vorgesehen, daß das Trägerrohr in Höhe des Rotors einen mit Drehstrom speisbaren Drehfeldstator aufweist und der Rotor einen radial magnetisierten Polrad-magneten aufweist. Hierdurch entsteht ein Synchronmotor, in dem durch Beaufschlagung des Drehfeldstators mit Drehstrom ein Drehfeld erzeugt werden kann, das den Rotor mitnimmt, so daß dem Rotor eine Drehbewegung aufgeprägt werden kann. Die Rotoreinrichtung erhält hierdurch motorische Eigenschaften und kann durch entsprechende Ausbildung der Mantelfläche des Rotors nicht nur zu Meßzwecken, sondern auch als Pumpe zur Förderung des Fluids verwendet werden. Vorzugsweise sollte der Polradmagnet wenigstens vier in unterschiedlichen radialen Richtungen magnetisierte Magnetsegmente aufweisen, damit Taumelbewegungen des drehenden Rotors aufgrund magnetischer Feldasymmetrien im Bereich der Lagerspalte zwischen Rotor und Radialstabilisatoren entgegengewirkt wird. Der Drehfeldstator sollte mit einem elektronischen Drehstromgenerator verbunden sein, auf den eine Lastwinkelregelung einwirkt. Durch gezielte Regelung des Lastwinkels können sowohl Betrag als auch Richtung des auf den Rotor wirkenden Drehfeldes bzw. Drehmoments eingestellt und stabilisiert werden.

Der Rotor ist an den jeweiligen Einsatzzweck angepaßt. So kann der Rotor flügelartige Vorsprünge aufweisen, wenn die erfindungsgemäße Rotoreinrichtung als Flügelrad- bzw. Turbinenradmeßeinrichtung für die Durchflußmessung eingesetzt werden soll. Sofern die Rotoreinrichtung in oben beschriebener Weise durch einen Synchronmotor erweitert ist, kann sie als Förderpumpe eingesetzt werden. Hierzu kann beispielsweise die Mantelfläche des Rotors mit wenigstens einem schraubenförmigen Steg versehen sein, so daß zwischen den einzelnen Stegwindungen Kanäle ausgebildet werden, die eine Förderung bewirken. Pumpen dieses Typs werden verbreitet zur Erzeugung von Hochvakuum eingesetzt.

Alternativ dazu können an der Mantelfläche des Rotors Schaufelkränze ausgebildet sein, die sich mit komplementären Schaufelkränzen an der Innenwandung des Trägerrohrs axial überschneiden. Je nach Ausbildung der Schaufelkränze ergeben sich die verschiedensten Einsatzmöglichkeiten, z.B. in Gasturbinen oder Hochvakuumpumpen. Die vorbeschriebene Schaufelkranzausbildung kann auch mit schraubenförmig verlaufenden Kanälen kombiniert werden. Man erhält auf diese Weise eine in der Vakuumtechnik unter der Bezeichnung "Compoundpumpe" bekannte Pumpe mit besonders hohem Kompressionsverhältnis.

Statt Schaufelkränzen und Stegen kann die Mantelfläche des Rotors auch glatt, insbesondere zylindrisch ausgebildet sein. Sofern die Rotoreinrichtung in oben beschriebener Weise einen Synchronmotor aufweist, kann die Viskosität gasförmiger oder flüssiger Medien dadurch gemessen werden, daß die elektrische Leistungsaufnahme des Synchronmotors zur Aufrechterhaltung einer bestimmten Rotordrehzahl gemessen wird. Diese ist zu der Reibleistung an der Mantelfläche des Rotors im wesentlichen proportional, wobei die Reibleistung ihrerseits ein Maß für die Viskosität des den Rotor umgebenden Mediums ist.

Nach einem weiteren Merkmal der Erfindung ist vorgeschlagen, daß die Mantelfläche des Rotors wenigstens einen radial nach außen vorstehenden Vorsprung aufweist und ein Sensor vorhanden ist, der die Axialposition des Rotors erfaßt und ein zur Axialposition proportionales Signal erzeugt. Der Vorsprung beispielsweise in Form eines Ringsteges bietet die Möglichkeit zur Messung der axialen Strömungsgeschwindigkeit eines gasförmigen oder flüssigen Mediums, indem die von der Strömung über den Vorsprung auf den Rotor übertragene Axialkraft über die entsprechende axiale Positionsverschiebung des Rotors ermittelt wird, indem die Axialstabilisiereinrichtung ein entsprechendes Signal erzeugt. Beide Maßnahmen können auch miteinander kombiniert werden, sofern die Rotoreinrichtung in oben beschriebener Weise mit einem Synchronmotor versehen ist. Dann können die Axialverschiebung des Rotors sowie die dem Rotor zugeführte Antriebsleistung mit Hilfe des Synchronmotors erfaßt werden, so daß sowohl die Strömungsgeschwindigkeit als auch die Viskosität des Fluids zeitgleich gemessen werden können.

In der Zeichnung ist die Erfindung anhand von Ausführungsbeispielen näher veranschaulicht. Es zeigen:
- Figur 1: einen Längsschnitt durch die erfindungsgemäße Rotoreinrichtung als Meßgerät;
- Figur 2: einen Längsschnitt durch die Kombination von Trägerrohr und Rotor in einer zweiten Ausführungsform für eine Pumpe;
- Figur 3: einen Längsschnitt durch die Kombination aus Rotor und Trägerrohr in einer dritten Ausführungsform für eine Pumpe;
- Figur 4: einen Längsschnitt durch die Kombination aus Trägerrohr und Rotor in einer vierten Ausführungsform für ein Meßgerät;
- Figur 5: einen Längsschnitt durch die Kombination aus Rotornabe und Radialstabilisatoren in einer fünften Ausführungsform.

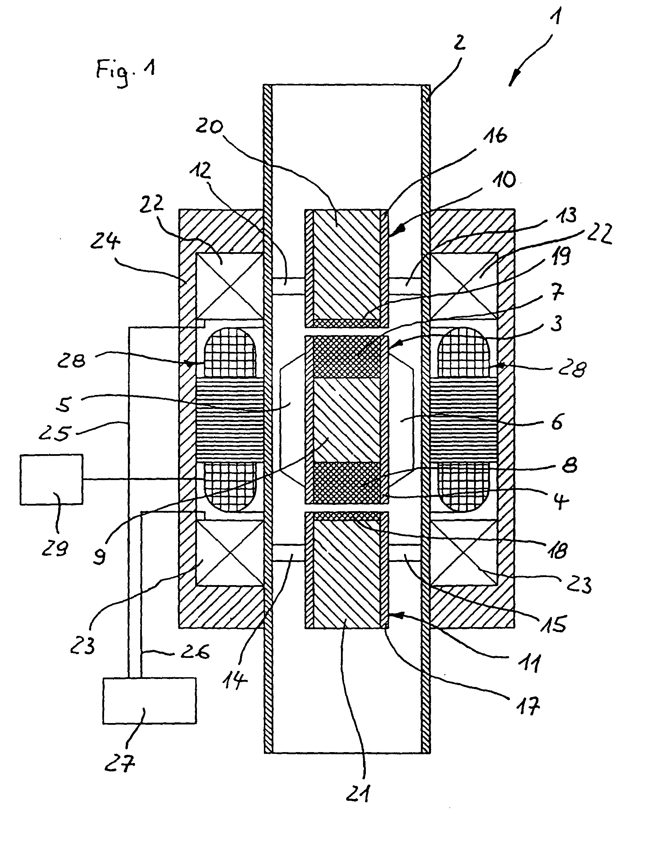

Figur 1 zeigt eine insgesamt mit 1 bezeichnete Rotoreinrichtung für den Einbau in ein flüssigkeits- oder gasführendes Rohr. Die Rotoreinrichtung 1 hat ein zylindrisches Trägerrohr 2, das über hier nicht näher dargestellte Flansche als zwischenstück in das Rohr einbaubar ist, so daß die Flüssigkeit oder das Gas das Trägerrohr 2 durchströmt.

In dem Trägerrohr 2 befindet sich mittig ein Rotor 3, der eine zylindrische Rotornabe 4 aufweist, an deren Außenseite Flügel 5, 6 angeformt sind. In den Endbereichen schließt die Rotornabe 4 zwei als Permanentmagnete ausgebildete Rotormagnete 7, 8 ein, die axial magnetisiert sind. Zwischen beiden Rotormagneten 7, 8 ist ein Polradmagnet 9 angeordnet, der über den Umfang verteilt in vier radialen Richtungen magnetisiert ist.

Benachbart zu den beiden Stirnseiten der Rotornabe 4 sind Radialstabilisatoren 10, 11 angeordnet, die über Stege 12, 13, 14, 15 derart an der Innenseite des Trägerrohrs 2 und koaxial zum Rotor 3 befestigt sind, daß sie axialsymmetrisch zum Rotor 3 liegen. Die Radialstabilisatoren 10, 11 weisen zylindrische Stabilisatorhülsen 16, 17 auf, deren Durchmesser dem der Rotornabe 4 entsprechen. Die Radialstabilisatoren 10, 11 bilden also hinsichtlich ihrer Außenkontur Fortsetzungen der Rotornabe 4. Die Stabilisatorhülsen 16, 17 schließen in den dem Rotor 3 benachbarten Bereich jeweils einen als Permanentmagnet ausgebildeten Statormagneten 18, 19 ein, wobei die Statormagnete 18, 19 derart axial magnetisiert sind, daß in den Spalten zwischen den Radialstabilisatoren 10, 11 und dem Rotor 3 ein axial gerichtetes, den Rotor 3 anziehendes Magnetfeld entsteht. Diese Magnetfelder sorgen dafür, daß der Rotor 3 immer mittig zur Achse des Trägerrohrs 2 gehalten wird, also eventuelle radiale Auslenkungen sofort wieder zurückgestellt werden. Dabei wird eine hohe Lagersteifigkeit in radialer Richtung erzielt.

Die Radialstabilisatoren 10 weisen zusätzliche ferromagnetische Flußleitstücke 20, 21 auf, die mit ringförmigen, elektrischen Magnetspulen 22, 23 einer Axialstabilisiereinrichtung zusammenwirken. Die Magnetspulen 22, 23 sind auf Höhe der Flußleitstücke 20, 21 angeordnet und umgeben das Trägerrohr 2 außenseitig. Sie sitzen innerhalb eines das Trägerrohr 2 ringförmig umgebenden Gehäuses 24, das in den stirnseitigen Bereichen gleichzeitig als Flußleitstück für die Magnetspulen 22, 23 dient. Die beiden Magnetspulen 22, 23 sind über Elektroleitungen 25, 26 mit einer Regeleinrichtung 27 verbunden. Die Regeleinrichtung 27 speist die Magnetspulen 22, 23 mit Erregerstrom. Hierdurch wird der magnetische Fluß in den Spalten zwischen Rotor 3 und den Radialstabilisatoren 10, 11 so überlagert und geregelt, daß der Rotor 3 eine allseitig berührungslose, axial stabile Position zwischen den Radialstabilisatoren 10, 11 einnimmt. Dabei werden die Magnetspulen 22, 23 nicht nur zur Flußregelung, sondern gleichzeitig auch als Sensorspulen für die berührungslose Abtastung der Axialposition des Rotors 3 verwendet, wie dies in gleicher Weise bei dem Magnetlager nach der DE-A-24 44 099 geschieht.

Zwischen den beiden Magnetspulen 22, 23 ist in dem Gehäuse 24 ein ringförmiger Drehfeldstator 28 angeordnet, der zusammen mit dem Polradmagnet 9 im Rotor 3 einen Synchronmotor bildet. Der Drehfeldstator 28 ist hierzu mit einem elektronischen Drehstromgenerator 29 verbunden. Dieser kann den Drehfeldstator 28 mit einem Drehstrom beaufschlagen, wobei mittels gezielter Regelung des Lastwinkels sowohl der Betrag als auch die Richtung des auf den Rotor 3 wirkenden Drehmoments eingestellt und stabilisiert werden kann.

Die vorbeschriebene Rotoreinrichtung 1 kann für verschiedene Zwecke eingesetzt werden. So kann sie zum Messen der Durchflußmenge von Flüssigkeiten und Gasen in Rohrleitungen verwendet werden. In diesem Fall sind die Flügel 5, 6 so ausgebildet, daß die in dem Ringkanal zwischen Rotor 3 und Trägerrohr 2 durchströmende Flüssigkeit den Rotor 3 in eine zur Geschwindigkeit der Flüssigkeit proportionalen Drehzahl versetzt, wobei die Durchströmgeschwindigkeit ein Maß für das Volumen des durchfließenden Mediums darstellt. Auch gasförmige Medien können gemessen werden. Die Messung der Drehzahl des Rotors 3 kann beispielsweise durch einen induktiven Impulsabgriff an dem Drehfeldstator 28 geschehen, wobei der Polradmagnet 9 den Impulsgeber bildet. Dabei wirkt sich für die Meßgenauigkeit vorteilhaft aus, daß der Rotor 3 des Magnetlagers 1 reibungslos gelagert ist und praktisch keinem Verschleiß ausgesetzt ist und somit auch keiner Wartung bedarf.

Sofern der Rotor 3 ohne Flügel 5, 6 ausgebildet ist, also eine glatte zylindrische Mantelfläche aufweist, kann die erfindungsgemäße Rotoreinrichtung 1 auch zur Messung der Viskosität gasförmiger bzw. flüssiger Medien eingesetzt werden. Zu diesem Zweck wird der Rotor 3 mit Hilfe des Drehstromgenerators 29 und des aus Drehfeldstator 28 und Polradmagnet 9 bestehenden Synchronmotors in Gang gesetzt, und es wird die elektrische Leistungsaufnahme des Synchronmotors zur Aufrechterhaltung einer bestimmten Rotordrehzahl gemessen. Diese ist der Reibleistung an der Mantelfläche des Rotors 3 im wesentlichen proportional. Die Reibleistung ihrerseits ist wieder ein Maß für die Viskosität des den Rotor 3 umgebenden Mediums.

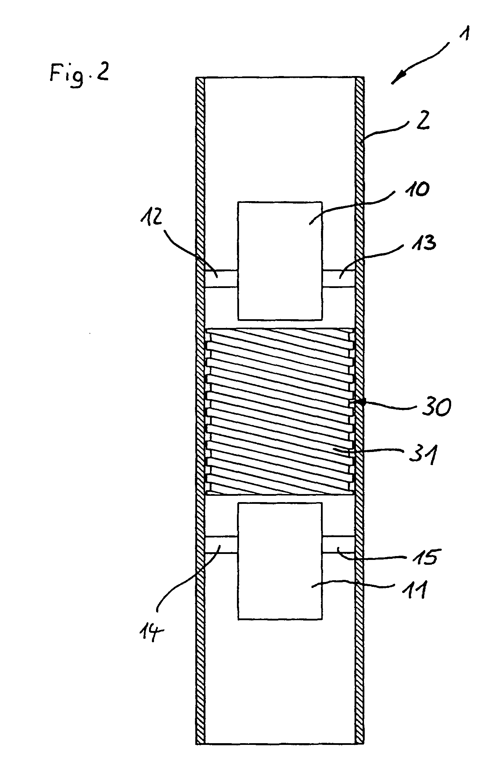

Die Ausführungsbeispiele gemäß den Figuren 2 bis 4 unterscheiden sich von dem gemäß Figur 1 lediglich durch andere Formgebungen des Rotors, wobei die außerhalb des Trägerrohrs 2 liegenden Teile mit dem Ausführungsbeispiel gemäß Figur 1 identisch sind und aus Übersichtlichkeitsgründen weggelassen sind. In den Figuren 2 bis 4 werden diejenigen Teile mit den schon für Figur 1 verwendeten Bezugsziffern versehen, die bei den Ausführungsbeispielen gemäß Figur 2 bis 4 die gleiche Ausbildung und/oder Funktion haben.

In Figur 2 hat der Rotor 30 einen Außendurchmesser, der nahezu dem Innendurchmesser des Trägerrohrs 2 entspricht. In seine Mantelfläche sind schraubenartig verlaufende Kanäle - beispielhaft mit 31 bezeichnet - eingeformt. Der Rotor 30 kann über den aus Drehfeldstator 28 und Polradmagnet 9 bestehenden Synchronmotor in Drehbewegung versetzt werden und wirkt dann als Förderpumpe. Eine solche Rotoreinrichtung 1 kann dann zur Erzeugung von Hochvakuum eingesetzt werden.

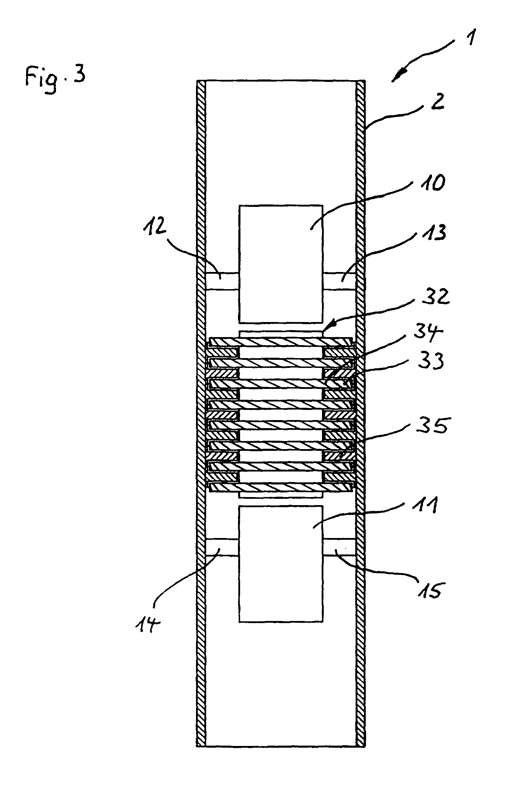

Bei dem Ausführungsbeispiel gemäß Figur 3 ist ein Rotor 32 mit insgesamt acht im Abstand zueinander angeordneten Schaufeklkränze - beispielhaft mit 33 bezeichnet - ausgebildet, die aus einer Vielzahl von Einzelschaufeln - beispielhaft mit 34 bezeichnet - bestehen. In die Zwischenräume zwischen den Schaufelkränzen 33 ragen an dem Trägerrohr 4 befestigte Schaufelkränze - beispielhaft mit 35 bezeichnet - hinein. Sie bestehen ebenfalls aus Einzelschaufeln. Die Schaufelkränze 33 und 35 bilden die strömungstechnischen Teile eines Turbokompressors. Durch Antrieb des Rotors 32 mittels des aus Drehfeldstator 28 und Polradmagnet 9 bestehenden Synchronmotors kann die Förderung eines gasförmigen Mediums nach Art eines Turbokompressors bewirkt werden.

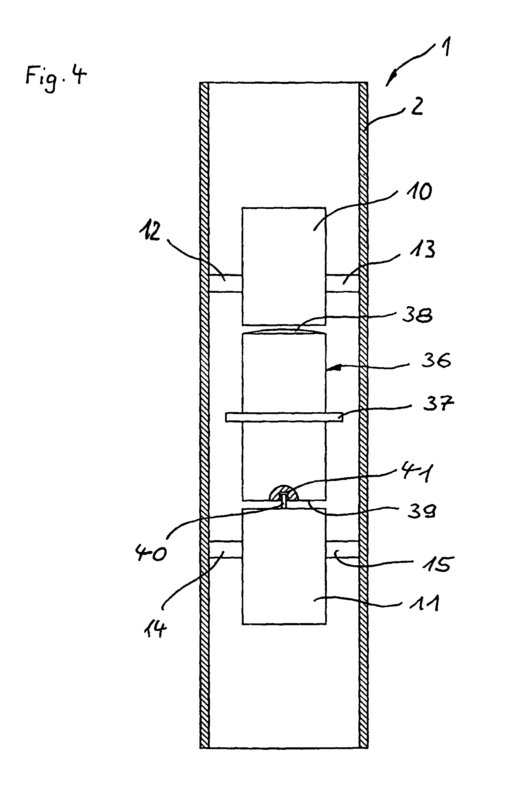

Bei dem Ausführungsbeispiel gemäß Figur 4 ist ein Rotor 36 vorgesehen, dessen Außenkontur sich von der des Rotors 3 gemäß Figur 1 nur dadurch unterscheidet, daß die im wesentlichen glatte zylindrische Mantelfläche in der axialen Mitte mit einem nach außen vorstehenden Ringsteg 37 versehen ist. Dieser Ringsteg 37 bildet einen Widerstand in der Strömung eines Mediums durch das Trägerrohr 2. Hierdurch wird auf den Rotor 36 eine Axialkraft übertragen, die zu einer entsprechenden axialen Positionsverschiebung des Rotors 36 führt. Diese wird über die Magnetspulen 22, 23 erfaßt und löst in der Regeleinrichtung 27 ein zur Axialverschiebung proportionales elektrisches Signal aus, das wiederum proportional zur Durchströmgeschwindigkeit ist.

Da die vom durchströmenden Medium auf den Rotor 36 ausgeübte Axialkraft nicht nur von der Strömungsgeschwindigkeit des Mediums, sondern auch von dessen Viskosität abhängig ist, ist es zweckmäßig, daß gleichzeitig auch die Viskosität des durchströmenden Mediums erfaßt wird. Dies geschieht - wie oben schon zu dem glatten Rotor 3 beschrieben - dadurch, daß der Rotor 36 mit Hilfe des Synchronmotors in eine bestimmte Drehbewegung versetzt wird und die hierfür erforderliche Antriebsleistung erfaßt und als Maß für die Viskosität des durchströmenden Mediums herangezogen wird.

Um ein mechanisches Anlaufen von achsfernen Bereichen des Rotors 36 zu vermeiden, hat er eine ballig ausgebildete Stirnseite 38. Sofern es zu einer Berührung zwischen Rotor 36 und Radialstabilisator 10 kommt, bleibt sie durch diese Ausbildung auf den Mittenbereich mit geringer Umfangsgeschwindigkeit begrenzt. Es versteht sich, daß auch die untere Stirnseite 39 entsprechend ballig ausgebildet sein kann.

Der untere Radialstabilisator 11 weist in der axialen Mitte an der dem Rotor 36 benachbarten Stirnseite einen Lagerstift 40 auf, der in eine Lagerausnehmung 41 im Rotor 36 einfaßt. Zwischen Lagerstift 40 und Lagerausnehmung 41 ist ein so großes Spiel, daß bei normalen radialen Abweichungen des Rotors 36 keine Berührung stattfindet. Nur wenn die radiale Auslenkung zu groß wird, verhindern Lagerstift 40 und Lagerausnehmung 41 eine weitere radiale Bewegung. Eine solche Radiallagerung kann selbstverständlich auch im Bereich des oberen Radialstabilisators 10 vorgesehen werden.

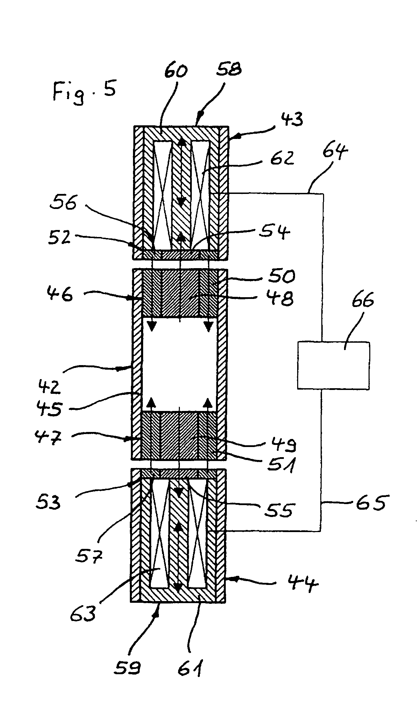

Bei dem Ausführungsbeispiel gemäß Figur 5 ist das Trägerrohr 2 mit allen daran angeordneten Teilen aus Gründen der Übersichtlichkeit weggelassen worden. Figur 5 zeigt einen Rotor 42, der zwischen zwei Radialstabilisatoren 43, 44 magnetisch gehalten ist. Der Rotor 42 hat eine außen glatte, im Querschnitt kreisrunde Rotornabe 45, deren Querschitt sich bei den Radialstabilisatoren 43, 44 fortsetzt. Nicht eingezeichnet sind die in Figur 1 dargestellten Stege 12, 13, 14, 15, mit denen die Radialstabilisatoren 43, 44 an der Innenseite des Trägerrohrs 2 befestigt sind.

Der Rotor 42 hat endseitig jeweils einen Rotormagneten 46, 47. Beide Rotormagnete 46, 47 sind zweiteilig aufgebaut. Sie bestehen jeweils aus einem zylinderförmigen Innenmagneten 48, 49 und einen diesen jeweils umgebenden, ringförmigen Außenmagneten 50, 51. Die Außenmagnete 50, 51 liegen mit ihren Innenseiten an den benachbarten Innenmagneten 48 bzw. 49 an.

Den Rotormagneten 46, 47 gegenüberliegend ist jeweils ein Statormagnet 52, 53. Auch die Statormagnete 52, 53 sind in der gleichen Weise zweiteilig aufgebaut wie die Rotormagnete 46, 47, d.h. sie haben jeweils einen zylinderförmigen Innenmagneten 54, 55 und einen diesen jeweils umgebenden, anliegenden und ringförmigen Außenmagneten 56, 57. Dabei entspricht der Durchmesser der Innenmagnete 54, 55 der Statormagnete 52, 53 dem Durchmesser der Innenmagnete 48, 49 der Rotormagnete 46, 47, während die Durchmesser der Außenmagnete 56, 57 der Statormagnete 52, 53 gleich den Durchmessern der Außenmagnete 50, 51 der Rotormagnete 46, 47 sind. Sie sind sämtlich koaxial angeordnet.

Die Rotormagnete 46, 47 und die Statormagnete 52, 53 sind derart magnetisiert, daß die jeweils benachbarten Paare der Magnete sich gegenseitig über ihre gesamte Fläche anziehen. Dabei besteht die Besonderheit des vorliegenden Ausführungsbeispiels darin, daß die sich jeweils paarweise gegenüberstehenden Innenmagnete 48, 54 bzw. 49, 55 axial vom Rotor 42 weg magnetisiert sind, während die sich jeweils paarweise gegenüberstehenden Außenmagnete 50, 56 bzw. 51, 57 axial in Richtung auf den Rotor 42, also entgegengesetzt, magnetisiert sind. Dies ist durch die Pfeile symbolisiert. Die entgegengesetzte Magnetisierung erhöht die Steifigkeit der Lagerung des Rotors 42 in radialer Richtung sehr wesentlich.

In den Radialstabilisatoren 43, 44 sind Topfspulen 58, 59 angeordnet, die jeweils aus einem topfförmigen ferromagnetischen Joch 60, 61 und einer darin angeordneten elektrischen Spule 62, 63 bestehen. Die Joche 60, 61 sind zu den Statormagneten 52, 53 hin offen. Der Mittendurchmesser der Spulen 62, 63 entspricht dem Außendurchmesser des jeweils benachbarten Innenmagneten 54, 55. Über elektrische Leitungen 64, 65 sind die Spulen 62, 63 mit einer Regeleinrichtung 66 verbunden. Die Regeleinrichtung 6 speist die Spulen 62, 63 mit Erregestrom. Je nach Richtung des Stroms kann der axiale Magnetfluß in dem Spalt zwischen Rotor 42 und Radialstabilisatoren 43, 44 verstärkt oder abgeschwächt werden, was durch den mittigen Doppelpfeil angedeutet ist. Hierdurch werden die dort wirksamen Axialkräfte in der Weise variiert, daß der Rotor 42 immer in der axialen Mittelstellung zwischen den Radialstabilisatoren 43, 44 geführt wird.

## Patentansprüche

1. Rotoreinrichtung (1) zur Interaktion eines Rotors (3, 30, 32, 36, 42) mit einem Fluid, mit folgenden Merkmalen:
a) die Rotoreinrichtung (1) hat ein Trägerrohr (2);
b) in dem Trägerrohr (2) ist ein Rotor (3, 30, 32, 36, 42) drehbar gelagert;
c) der Rotor (3, 30, 32, 36, 42) ist für die Interaktion mit dem durch das Trägerrohr (2) strömenden Fluid ausgebildet;
d) der Rotor (3, 30, 32, 36, 42) weist an beiden Stirnseiten axial magnetisierte permanentmagnetische Rotormagnete (7, 8; 46, 47) auf;
e) den Stirnseiten des Rotors (3, 30, 32, 36, 42) axial unmittelbar gegenüberliegend sind mit dem Trägerrohr (2) verbundene, permanentmagnetische Statormagnete (18, 19; 52, 53) angeordnet;
f) jeder Statormagnet (18, 19; 52, 53) hat eine solche axiale Magnetisierung, daß sich benachbarte Stator- und Rotormagnete (18, 19; 7, 8; 46, 47; 52, 53) gegenseitig anziehen.
g) die Rotoreinrichtung (1) weist eine magnetische Axialstabilisiereinrichtung (22, 23, 27; 58, 59, 66) für den Rotor (3, 30, 32, 36, 42) auf.

2. Rotoreinrichtung nach Anspruch 1, **dadurch gekennzeichnet, daß** die Rotor- und Statormagnete (46, 47; 52, 53) jeweils aus mindestens zwei ineinandergefügten Teilmagneten (48, 49, 50, 51; 54, 55, 56, 57) bestehen, wobei jeweils radial benachbarte Teilmagnete (48, 49, 50, 51; 54, 55, 56, 57) entgegengesetzt magnetisiert sind.

3. Rotoreinrichtung (1) zur Interaktion eines Rotors (3, 30, 32, 36, 42) mit einem Fluid, mit folgenden Merkmalen:
a) die Rotoreinrichtung (1) hat ein Trägerrohr (2);
b) in dem Trägerrohr (2) ist ein Rotor (3, 30, 32, 36, 42) drehbar gelagert;
c) der Rotor (3, 30, 32, 36, 42) ist für die Interaktion mit dem durch das Trägerrohr (2) strömenden Fluid ausgebildet;
d) an den Stirnseiten des Rotors (3, 30, 32, 36, 42) stehen sich jeweils unmittelbar ein axial magnetisierter, permanent magnetischer Magnet und ein Flußleitstück gegenüber, wobei der Magnet entweder als Rotormag-net (7, 8; 46, 47) am Rotor (3, 30, 32, 36, 42) sitzt oder als Statormagnet (18, 19; 52, 53) mit dem Trägerrohr (2) verbunden ist;
e) die Rotoreinrichtung (1) weist eine magnetische Axialstabilisiereinrichtung (22, 23, 27; 58, 59, 66) für den Rotor (3, 30, 32, 36, 42) auf.

4. Rotoreinrichtung nach Anspruch 3, **dadurch gekennzeichnet, daß** elektrische Magnetspulen zur Verstärkung der Magnetisierung der Flußleitstücke im Sinne einer Vergrößerung der Anziehungskräfte zwischen den Magneten (7, 8; 46, 47) und den Flußleitstücken vorgesehen sind.

5. Rotoreinrichtung nach einem der Ansprüche 1 bis 4, **dadurch gekennzeichnet, daß** der Rotor (3, 30, 32, 36, 42) als Axialrotor ausgebildet ist.

6. Rotoreinrichtung nach einem der Ansprüche 1 bis 5, **dadurch gekennzeichnet, daß** der Rotor (3, 30, 32, 36, 42) eine Rotornabe (45) aufweist und die Rotormagnete (7, 8; 46, 47) bzw. die Flußleitstücke in der Rotornabe (45) angeordnet sind, wobei die Statormagnete (18, 19; 52, 53) bzw. die Flußleitstücke den Stirnseiten der Rotornabe (45) gegenüberliegend angeordnet sind.

7. Rotoreinrichtung nach Anspruch 6, **dadurch gekennzeichnet, daß** die Statormagnete (18, 19; 52, 53) bzw. die Flußleitstücke in Radialstabilisatoren (10, 11; 43, 44) angeordnet sind, deren Kontur nicht über die der Rotornabe (45) vorsteht.

8. Rotoreinrichtung nach Anspruch 7, **dadurch gekennzeichnet, daß** von den jeweils gegenüberliegenden Stirnseiten der Radialstabilisatoren (10, 11) und des Rotors (36) zumindest jeweils eine sphärisch ausgebildet ist.

9. Rotoreinrichtung nach Anspruch 7 oder 8, **dadurch gekennzeichnet, daß** die jeweils gegenüberliegenden Stirnflächen der Radialstabilisatoren (10, 11) und des Rotors (36) mit ineinandergreifenden komplentären Lagerzapfen (40) und Lagerausnehmungen (41) versehen sind, die die Radial- und/oder Axialbeweglichkeit des Rotors (36) begrenzen.

10. Rotoreinrichtung nach einem der Ansprüche 1 bis 9, **dadurch gekennzeichnet, daß** die Rotormagnete (7, 8; 46, 47) bzw. Flußleitstücke und die Statormagnete (18, 19; 52, 53) bzw. Flußleitstücke unmittelbar gegenüberliegend angeordnet sind.

11. Rotoreinrichtung nach einem der Ansprüche 1 bis 10, **dadurch gekennzeichnet, daß** die Axialstabilisiereinrichtung wenigstens eine elektrische Magnetspule (22, 23; 58, 59) sowie eine Regeleinrichtung (27, 66) mit einem die Axialbewegung des Rotors (3, 30, 36, 42) erfassenden Sensor aufweist, wobei die Regeleinrichtung (27, 66) den elektrischen Stromfluß in der Magnetspule bzw. den Magnetspulen (22, 23; 58, 59) derart beeinflußt, daß das Magnetfeld der Magnetspule(n) (22, 23; 58, 59) einer Axialbewegung des Rotors (3, 30, 32, 36, 42) aus seiner Sollage entgegenwirkt.

12. Rotoreinrichtung nach Anspruch 11, **dadurch gekennzeichnet, daß** die Axialstabilisiereinrichtung zwei Magnetspulen (22, 23; 58, 59) aufweist, die im Bereich der Statormagnete (18, 19; 52, 53) bzw. Flußleitstücke und/oder der Stirnflächen des Rotors (3, 30, 32, 36, 42) angeordnet sind.

13. Rotoreinrichtung nach Anspruch 12, **dadurch gekennzeichnet, daß** die Magnetspulen (22, 23) das Trägerrohr (2) umgeben.

14. Rotoreinrichtung nach Anspruch 12, **dadurch gekennzeichnet, daß** die Magnetspulen (58, 59) in den Radialstabilisatoren (43, 44) angeordnet sind.

15. Rotoreinrichtung nach einem der Ansprüche 11 bis 14, **dadurch gekennzeichnet, daß** die Radialstabilisatoren (10, 11; 43, 44) magnetisierbare Flußleitstücke (20, 21; 60, 61) in einer solchen Ausbildung und Anordnung aufweisen, daß das axiale Magnetfeld im Spalt zwischen den Stirnseiten von Radialstabilisatoren (10, 11; 43, 44) und Rotor (3, 30, 32, 36, 42) durch das von den Magnetspulen (22, 23; 62, 63) erzeugte Magnetfeld in axialer Richtung überlagert wird.

16. Rotoreinrichtung nach Anspruch 15, **dadurch gekennzeichnet, daß** die Flußleitstücke (20, 21; 60, 61) auf Höhe der Magnetspulen (22, 23; 62, 63) angeordnet sind.

17. Rotoreinrichtung nach einem der Ansprüche 1 bis 16, **dadurch gekennzeichnet, daß** der Rotor (3, 30, 32, 36, 42) einen Impulsgeber (9) und das Trägerrohr (2) einen Impulsnehmer (28) aufweist und daß der Impulsgeber (9) für den Impulsnehmer (28) der Drehzahl des Rotors (3, 30, 32, 36, 42) entsprechende Impulse erzeugt.

18. Rotoreinrichtung nach Anspruch 17, **dadurch gekennzeichnet, daß** der Impulsgeber als Impulsmagnet(e) (9) und der Impulsnehmer als Spule (28) ausgebildet sind.

19. Rotoreinrichtung nach einem der Ansprüche 1 bis 18, **dadurch gekennzeichnet, daß** das Trägerrohr (2) in Höhe des Rotors (3, 30, 32, 36, 42) einen mit Drehstrom speisbaren Drehfeldstator (28) aufweist und der Rotor (3, 30, 32, 36, 42) einen radial magnetisierten Polradmagneten (9) aufweist.

20. Rotoreinrichtung nach Anspruch 19, **dadurch gekennzeichnet, daß** der Polradmagnet (9) wenigstens vier in unterschiedlichen Radialrichtungen magnetisierte Magnetsegmente aufweist.

21. Rotoreinrichtung nach Anspruch 19 oder 20, **dadurch gekennzeichnet, daß** der Drehfeldstator (28) mit einem elektronischen Drehstromgenerator (29) verbunden ist, auf den vorzugsweise eine Lastwinkelregelung einwirkt.

22. Rotoreinrichtung nach einem der Ansprüche 1 bis 21, **dadurch gekennzeichnet, daß** wenigstens ein schraubenförmiger Steg zwecks Bildung schraubenförmiger Kanäle (31) an der Mantelfläche des Rotors (30) und/oder am Trägerrohr (2) vorgesehen ist.

23. Rotoreinrichtung nach einem der Ansprüche 1 bis 21, **dadurch gekennzeichnet, daß** an der Mantelfläche des Rotors (32) Schaufelkränze (33) ausgebildet sind und sich diese mit komplementären Schaufelkränzen (35) an der Innenwandung des Trägerrohrs (2) axial überschneiden.

24. Rotoreinrichtung nach einem der Ansprüche 1 bis 23, **dadurch gekennzeichnet, daß** die Mantelfläche des Rotors (42) glatt, insbesondere zylindrisch ausgebildet ist.

25. Rotoreinrichtung nach einem der Ansprüche 1 bis 24, **dadurch gekennzeichnet, daß** die Mantelfläche des Rotors (36) wenigstens einen radial nach außen vorstehenden Vorsprung (37) aufweist und ein Sensor vorhanden ist, der die Axialposition des Rotors (36) erfaßt und ein zur Axialposition proportionales Signal erzeugt.

## Claims

1. Rotor device (1) for interaction of a rotor (3, 30, 32, 36, 42) with a fluid, which has the following features:
a) the rotor device (1) has a support tube (2);
b)a rotor (3, 30, 32, 36, 42) is supported within the support tube (2) so as to be able to rotate;
c) the rotor (3, 30, 32, 36, 42) is configured to interact with the fluid flowing through the support tube (2);
d) at both its end faces, the rotor (3, 30, 32, 36, 42) has axially magnetized rotor magnets (7, 8; 46; 47) that are permanent magnets
e) axially opposite the end faces of the rotor (3, 30, 32, 36, 42) there are permanent magnet stator magnets (18, 19; 52, 53) that are connected to the support tube (2) and are axially opposite each other;
f) each stator magnet (18, 19; 52, 53) is axially magnetized in such a way that the adjacent stator magnets and rotor magnets (18, 19; 7, 8; 46, 47; 52, 53) attract each other;
g) the rotor device (1) has a magnetic axial stabilizer device (22, 23, 27; 58, 59, 66) for the rotor (3, 30, 32, 36, 42).

2. Rotor device as defined in Claim 1, **characterized in that** the rotor magnets and stator magnets (46, 47; 52, 53) each comprise at least two magnets (48, 49, 50, 51; 54, 55, 56, 57) that fit into each other, each pair of radially adjacent magnets (48, 49, 50, 51; 54, 55, 56, 57) being magnetized with the opposite polarity.

3. Rotor device (1) for interaction of a rotor (3, 30, 32, 36, 42) with a fluid, which has the following features:
a) the rotor device (1) has a support tube (2);
b) a rotor (3, 30, 32, 36, 42) is supported within the support tube (2) so as to be able to rotate;
c)the rotor (3, 30, 32, 36, 42) is configured to interact with the fluid flowing through the support tube (2);
d) in each instance an axially magnetized permanent magnet and a flux deflector are opposite the end faces of the rotor (3, 30, 32, 36, 42), the magnet being either a rotor magnet installed on the rotor (3, 30, 32, 36, 42) as a rotor magnet (7, 8; 46, 47) or as a stator magnet (18, 19; 52, 53) that is connected to the support tube (2);
e) the rotor device (1) has a magnetic axial stabilizer device (22, 23, 27; 58, 59, 66) for the rotor (3, 30, 32, 36, 42).

4. Rotor device as defined in Claim 3, **characterized in that** electrical field coils are provided so as to increase the magnetization of the flux deflectors, in the sense that they amplify the attractive force between the magnets (7, 8; 46, 47) and the flux deflectors.

5. Rotor device as defined in one of the Claims 1 to 4, **characterized in that** the rotor (3, 30, 32, 36, 42) is configured as an axial rotor.

6. Rotor device as defined in one of the Claims 1 to 5, **characterized in that** the rotor (3, 30, 32, 36, 42) has a rotor hub (45) and the rotor magnets (7, 8; 46, 47) or the flux deflectors are arranged within the rotor hub (45), the stator magnets (18, 19; 52, 53) or the flux deflectors being arranged so as to be opposite the end faces of the rotor hub (45).

7. Rotor device as defined in Claim 6, **characterized in that** the stator magnets (18, 19; 52, 53) or the flux deflectors are arranged in the radial stabilizers (10, 11; 43, 44), and their outlines do not extend beyond the rotor hub (45).

8. Rotor device as defined in Claim 7, **characterized in that** of the end faces of the radial stabilizers (10, 11) and of the rotor (36) that are opposite each other, at least one is configured so as to be spherical.

9. Rotor device as defined in Claim 7 or Claim 8, **characterized in that** the opposing end faces of the radial stabilizers (10, 11) and of the rotor (36) are provided with complementary bearing pins (40) and bearing recesses (41) that fit into each other, that limit the radial and/or the axial mobility of the rotor (36).

10. Rotor device as defined in one of the Claims 1 to 9, **characterized in that** the rotor magnets (7, 8; 46, 47) or flux deflectors and the stator magnets (18, 19; 52, 53) or flux deflectors are arranged so as to be directly opposite each other.

11. Rotor device as defined in one of the Claims 1 to 10, **characterized in that** the axial stabilizing device incorporates at least one electrical field coil (22, 23; 58, 59) as well as a regulating device (27, 66) with a sensor that detects the axial movement of the rotor ((3, 30, 32, 36, 42), the regulating device (27, 66) influencing the flow of electrical current in the field coil or field coils (22, 23; 58, 59) in such a way that the magnetic field of the field coil(s) (22, 23; 58, 59) counteracts any axial movement of the rotor (3, 30, 32, 36, 42) out of its intended position.

12. Rotor device as defined in Claim 11, **characterized in that** the axial stabilizing device incorporates two field coils (22, 23; 58, 59) that are arranged in the vicinity of the stator magnets (18, 19; 52, 53) or flux def lectors and/or the end faces of the rotor ((3, 30, 32, 36, 42).

13. Rotor device as defined in Claim 12, **characterized in that** the field coils (22, 23) surround the support tube (2).

14. Rotor device as defined in Claim 12, **characterized in that** the field coils (58, 59) are arranged in the radial stabilizers (43, 44).

15. Rotor device as defined in one of the Claims 11 to 14, **characterized in that** the radial stabilizers (10, 11; 43, 44) incorporate magnetizable flux deflectors (20, 21; 60, 61) of such a configuration and in such an arrangement that the axial magnetic field in the gap between the end faces of the radial stabilizers (10, 11; 43, 44) and the rotor (3, 30, 32, 36, 42) are overlaid in an axial direction by the magnetic field generated by the field coils (22, 23; 62, 63).

16. Rotor device as defined in Claim 15, **characterized in that** the flux deflectors (20, 21; 60, 61) are arranged at the level of the field coils (22, 23; 62, 63).

17. Rotor device as defined in one of the Claims 1 to 16, **characterized in that** the rotor (3, 30, 32, 36, 42) incorporates a pulse emitter (9) and the support tube (2) incorporates a pulse receiver ( 28 ) ; and **in that** the pulse emitter (9) generates pulses that correspond to the speed of rotation of the rotor (3, 30, 32, 36, 42) for the pulse receiver (28).

18. Rotor device as defined in Claim 17, **characterized in that** the pulse emitter is configured as a pulse magnet or pulse magnets (9) and the pulse receiver is configured as coil (28).

19. Rotor device as defined in one of the Claims 1 to 18, **characterized in that** the support tube (2) has, as the level of the rotor (3, 30, 32, 36, 42) a rotary field stator (28) that can be supplied with rotary current, and the rotor (3, 30, 32, 36, 42) has a revolving field magnet (9) that can be magnetized radially.

20. Rotor device as defined in Claim 19, **characterized in that** the revolving field magnet (9) has at least four magnet segments that are magnetized in different radial directions.

21. Rotor device as defined in Claim 19 or Claim 20, **characterized in that** the rotary field stator (28) is connected to an electronic rotary current generator (29), it being preferred that a load-angle regulator act on this.

22. Rotor device as defined in one of the Claims 1 to 21, **characterized in that** at least one helical web is provided in order to form helical channels (31) on the outside surface of the rotor (30) and/or on the support tube (2).

23. Rotor device as defined in one of the Claims 1 to 21, **characterized in that** blade rings (33) are formed on the outside surface of the rotor (32) and that these intersect axially with complementary blade rings (35) on the inside wall of the support tube (2).

24. Rotor device as defined in one of the Claims 1 to 23, **characterized in that** the outside surface of the rotor (42) is smooth and, in particular, cylindrical.

25. Rotor device as defined in one of the Claims 1 to 24, **characterized in that** the outside surface of the rotor (36) has at least one projection (37) that extends radially outward, and has a sensor that detects the axial position of the rotor (36) and generates a signal that is proportional to the axial position.

## Revendications

1. Dispositif à rotor (1) pour l'interaction d'un rotor (3, 30, 32, 36, 42) avec un fluide, présentant les caractéristiques suivantes :
a) le dispositif à rotor (1) possède un tube-support (2) ;
b) un rotor (3, 30, 32, 36, 42) est monté à rotation dans le tube-support (2) ;
c) le rotor (3, 30, 32, 36, 42) est conçu pour interagir avec le fluide qui passe à travers le tube-support (2) ;
d) le rotor (3, 30, 32, 36, 42) présente aux deux extrémités des aimants de rotor (7, 8 ; 46, 47) à aimantation permanente et magnétisation axiale ;
e) des aimants de stator (18, 19 ; 52, 53) à aimantation permanente, solidaires du tube-support (2), sont disposés directement en face, en direction axiale, des extrémités du rotor (3, 30, 32, 36, 42) ;
f) chaque aimant de stator (18, 19 ; 52, 53) possède une magnétisation axiale telle que des aimants de stator et de rotor voisins (18, 19 ; 7, 8 ; 46, 47 ; 52, 53) s'attirent mutuellement ;
g) le dispositif à rotor (1) présente un dispositif magnétique de stabilisation axiale (22, 23, 27 ; 58, 59, 66) pour le rotor (3, 30, 32, 36, 42).

2. Dispositif à rotor selon la revendication 1, **caractérisé par le fait que** les aimants de rotor et de stator (46, 47 ; 52, 53) sont composés chacun d'au moins deux sous-aimants emboîtés l'un dans l'autre (48, 49, 50, 51 ; 54, 55, 56, 57), des sous-aimants voisins en direction radiale (48, 49, 50, 51 ; 54, 55, 56, 57) étant magnétisés en sens contraire.

3. Dispositif à rotor (1) pour l'interaction d'un rotor (3, 30, 32, 36, 42) avec un fluide, présentant les caractéristiques suivantes :
a) le dispositif à rotor (1) possède un tube-support (2) ;
b) un rotor (3, 30, 32, 36, 42) est monté rotatif dans le tube-support (2) ;
c) le rotor (3, 30, 32, 36, 42) est conçu pour interagir avec le fluide qui passe à travers le tube-support (2) ;
d) aux extrémités du rotor (3, 30, 32, 36, 42) se font face directement un aimant permanent à magnétisation axiale et un élément conducteur de flux, l'aimant étant soit un aimant de rotor (7, 8 ; 46, 47) monté sur le rotor (3, 30, 32, 36, 42), soit un aimant de stator (18, 19 ; 52, 53) solidaire du tube-support (2) ;
e) le dispositif à rotor (1) présente un dispositif magnétique de stabilisation axiale (22, 23, 27 ; 58, 59, 66) pour le rotor (3, 30, 32, 36, 42).

4. Dispositif à rotor selon la revendication 3, **caractérisé par le fait qu'**il est prévu des bobines d'excitation électriques pour amplifier la magnétisation des éléments conducteurs de flux en vue d'augmenter les forces d'attraction entre les aimants (7, 8 ; 46, 47) et les éléments conducteurs de flux.

5. Dispositif à rotor selon l'une des revendications 1 à 4, **caractérisé par le fait que** le rotor (3, 30, 32, 36, 42) est conçu comme un rotor axial.

6. Dispositif à rotor selon l'une des revendications 1 à 5, **caractérisé par le fait que** le rotor (3, 30, 32, 36, 42) présente un moyeu de rotor (45) et que les aimants de rotor (7, 8 ; 46, 47) ou les éléments conducteurs de flux sont disposés dans le moyeu de rotor (45), les aimants de stator (18, 19 ; 52, 53) ou les éléments conducteurs de flux étant disposés en face des extrémités du moyeu de rotor (45).

7. Dispositif à rotor selon la revendication 6, **caractérisé par le fait que** les aimants de stator (18, 19 ; 52, 53) ou les éléments conducteurs de flux sont disposés dans des stabilisateurs radiaux (10, 11; 43, 44) dont le contour ne dépasse pas celui du moyeu de rotor (45).

8. Dispositif à rotor selon la revendication 7, **caractérisé par le fait que** des extrémités en regard des stabilisateurs radiaux (10, 11) et du rotor (36), au moins une à chaque fois est de forme sphérique.

9. Dispositif à rotor selon la revendication 7 ou 8, **caractérisé par le fait que** les surfaces frontales en regard des stabilisateurs radiaux (10, 11) et du rotor (36) sont pourvues de tourillons (40) et d'évidements (41) complémentaires, s'emboîtant les uns dans les autres, qui limitent la mobilité radiale et axiale du rotor (36).

10. Dispositif à rotor selon l'une des revendications 1 à 9, **caractérisé par le fait que** les aimants de rotor (7, 8 ; 46, 47) ou les éléments conducteurs de flux et les aimants de stator (18, 19 ; 52, 53) ou les éléments conducteurs de flux sont disposés directement en face les uns des autres.

11. Dispositif à rotor selon l'une des revendications 1 à 10, **caractérisé par le fait que** le dispositif de stabilisation axiale présente au moins une bobine d'excitation électrique (22, 23 ; 58, 59) ainsi qu'un dispositif de réglage (27, 66) avec un capteur détectant le mouvement axial du rotor (3, 30, 32, 36, 42), le dispositif de réglage (27, 66) influant sur le courant électrique dans la ou les bobine(s) d'excitation (22, 23 ; 58, 59) de telle manière que le champ magnétique de la ou des bobine(s) d'excitation (22, 23 ; 58, 59) s' oppose à un déplacement axial du rotor (3, 30, 32, 36, 42) de sa position nominale.

12. Dispositif à rotor selon la revendication 11, **caractérisé par le fait que** le dispositif de stabilisation axiale présente deux bobines d'excitation (22, 23 ; 58, 59) qui sont disposées au niveau des aimants de stator (18, 19 ; 52, 53) ou des éléments conducteurs de flux et/ou des surfaces frontales du rotor (3, 30, 32, 36, 42).

13. Dispositif à rotor selon la revendication 12, **caractérisé par le fait que** les bobines d'excitation (22, 23) entourent le tube-support (2).

14. Dispositif à rotor selon la revendication 12, **caractérisé par le fait que** les bobines d'excitation (58, 59) sont disposées dans les stabilisateurs radiaux (43, 44).

15. Dispositif à rotor selon l'une des revendications 11 à 14, **caractérisé par le fait que** les stabilisateurs radiaux (10, 11 ; 43, 44) présentent des éléments conducteurs de flux magnétisables (20, 21 ; 60, 61) d'une forme et d'une disposition telles que le champ magnétique axial dans l'espace entre les extrémités des stabilisateurs radiaux (10, 11 ; 43, 44) et le rotor (3, 30, 32, 36, 42) est recouvert par le champ magnétique généré en direction axiale par les bobines d'excitation (22, 23 ; 62, 63).

16. Dispositif à rotor selon la revendication 15, **caractérisé par le fait que** les éléments conducteurs de flux (20, 21 ; 60, 61) sont disposés à hauteur des bobines d'excitation (22, 23 ; 62, 63).

17. Dispositif à rotor selon l'une des revendications 1 à 16, **caractérisé par le fait que** le rotor (3, 30, 32, 36, 42) présente un générateur d'impulsions (9) et le tube-support (2) un récepteur d'impulsions (28) et que le générateur d'impulsions (9) génère pour le récepteur d'impulsions (28) un nombre d'impulsions proportionnel à la vitesse de rotation du rotor (3, 30, 32, 36, 42).

18. Dispositif à rotor selon la revendication 17, **caractérisé par le fait que** le générateur d'impulsions (9) est constitué par un/des aimant(s) pulsé(s) (9) et le récepteur d'impulsions par une bobine (28).

19. Dispositif à rotor selon l'une des revendications 1 à 18, **caractérisé par le fait que** le tube-support (2) présente à hauteur du rotor (3, 30, 32, 36, 42) un stator à champ tournant (28) pouvant être alimenté en courant triphasé et que le rotor (3, 30, 32, 36, 42) présente un aimant à roue polaire (9) à magnétisation radiale.

20. Dispositif à rotor selon la revendication 19, **caractérisé par le fait que** l'aimant à roue polaire (9) présente au moins quatre segments d'aimant magnétisés dans des directions radiales différentes.

21. Dispositif à rotor selon la revendication 19 ou 20, **caractérisé par le fait que** le stator à champ tournant (28) est relié à un alternateur électronique à courant triphasé (29) sur lequel agit de préférence un régulateur d'angle de charge.

22. Dispositif à rotor selon l'une des revendications 1 à 21, **caractérisé par le fait qu'**il est prévu au moins une nervure hélicoïdale pour former des canaux hélicoïdaux (31) sur la surface d' enveloppe du rotor (30) et/ou sur le tube-support (2).

23. Dispositif à rotor selon l'une des revendications 1 à 21, **caractérisé par le fait qu'**il est formé des couronnes d'aubes (33) sur la surface d'enveloppe du rotor (32) et que celles-ci se recouvrent axialement avec des couronnes d'aubes complémentaires (35) sur la paroi interne du tube-support (2).

24. Dispositif à rotor selon l'une des revendications 1 à 23, **caractérisé par le fait que** la surface d'enveloppe du rotor (42) est lisse, en particulier de forme cylindrique.

25. Dispositif à rotor selon l'une des revendications 1 à 24, **caractérisé par le fait que** la surface d'enveloppe du rotor (36) présente au moins une partie en saillie (37) dépassant radialement vers l'extérieur et qu'il est prévu un capteur qui détecte la position axiale du rotor (36) et génère un signal proportionnel à la position axiale.
